# EUROPÄISCHE PATENTSCHRIFT

(11) **EP 3 227 672 B1**
(45) Veröffentlichungstag und Bekanntmachung des Hinweises auf die Patenterteilung: **06.03.2019**
(21) Anmeldenummer: 15775733.7
(22) Anmeldetag: 09.10.2015
(51) Int. Cl.: G01N 27/407, G01N 27/18, G01N 27/417, G01N 1/22, G01N 33/00

(54) **VERFAHREN ZUM HERSTELLEN EINER GASSENSORVORRICHTUNG ZUM ERFASSEN ZUMINDEST EINES GASFÖRMIGEN ANALYTEN**
METHOD FOR PRODUCING A GAS SENSOR DEVICE FOR DETECTING OF AT LEAST ONE GASEOUS ANALYTE
PROCÉDÉ DE FABRICATION D'UN DISPOSITIF CAPTEUR DE GAZ POUR DÉTECTER D'AU MOINS UN ANALYTE GAZEUX

(30) Priorität: 02.12.2014 DE 102014224587
(43) Veröffentlichungstag der Anmeldung: 11.10.2017
(73) Patentinhaber: Robert Bosch GmbH, 70442 Stuttgart (DE)
(72) Erfinder: WIDENMEYER, Markus, 71101 Schoenaich (DE); LETSCH, Andreas, 70565 Stuttgart (DE); KUNZ, Denis, 74199 Untergruppenbach (DE); ROELVER, Robert, 75365 Calw-Stammheim (DE)
(86) Internationale Anmeldenummer: PCT/EP2015/073463
(87) Internationale Veröffentlichungsnummer: WO 2016/087100

(56) Entgegenhaltungen:
- DE-A1-102013 204 197
- DE-A1-102013 204 665
- DE-A1-102013 208 939
- US-A1- 2013 075 255

## Beschreibung

### Stand der Technik

Die vorliegende Erfindung bezieht sich auf ein Verfahren zum Herstellen einer Gassensorvorrichtung zum Erfassen zumindest eines gasförmigen Analyten.

Beispielsweise können miniaturisierte Feststoffelektrolyt-Gassensoren, die unter Einsatz mikromechanischer Verfahren und Prozesse gefertigt sein können, insbesondere zur Messung eines Restsauerstoffanteils im Abgasstrom von Verbrennungsmotoren bzw. zur sogenannten Lambda-Messung oder dergleichen eingesetzt werden.

Aus der DE 10 2013 204 197 A1 sind ein mikroelektrochemischer Sensor sowie ein Verfahren zum Betreiben eines mikroelektrochemischen Sensors bekannt. Aus der US 2013/0075255 A1 ist ein elektrochemischer Sensor in MEMS-Technologie bekannt.

### Offenbarung der Erfindung

Vor diesem Hintergrund werden mit dem hier vorgestellten Ansatz ein Verfahren zum Herstellen einer Gassensorvorrichtung zum Erfassen zumindest eines gasförmigen Analyten gemäß dem Hauptanspruch vorgestellt. Vorteilhafte Ausgestaltungen ergeben sich aus den jeweiligen Unteransprüchen und der nachfolgenden Beschreibung.

Gemäß Ausführungsformen der vorliegenden Erfindung kann ein mikroelektrochemischer Sensor (MECS) auf Feststoffelektrolytbasis bzw. ein miniaturisierter Feststoffelektrolyt-Gassensor bereitgestellt werden, bei dem insbesondere auch im Falle extremer Oberflächentopografien, beispielsweise mit tiefen Kavitäten, eine Kombination von nasschemisch abgeschiedenen, funktionalen Sensorelektroden und trockenchemisch abgeschiedenen Elektroden zur elektrischen Weiterleitung des Sensorsignals realisiert werden kann. Das Verfahren kann in adaptierter Weise auch bei anderen Anwendungen bzw. zur Herstellung anderer Vorrichtungen ausgeführt werden, um strukturierte Schichten auf stark topografisch geformten Oberflächen bzw. nasschemisch aufgebrachte Metallelektroden auf einer topografisch anspruchsvollen Oberfläche zu erzeugen.

Gemäß den Ausführungsformen der vorliegenden Erfindung wird zum Einen ermöglicht, auch an topografisch anspruchsvollen Oberflächen ursprünglich geschlossene Schichten bzw. Filme lokal und selektiv zu strukturieren bzw. Schichtmaterial gezielt zu entfernen, um so beispielsweise nasschemisch abgeschiedene, funktionale Sensorelektroden und trockenchemisch abgeschiedene Elektroden zur elektrischen Weiterleitung des Sensorsignals miteinander zu kombinieren. Hierbei wird eine Laserablation zur selektiven Entschichtung von Filmen auf tiefenstrukturierten Oberflächen eingesetzt. Auch wird zum Anderen vorteilhafterweise gemäß Ausführungsformen der vorliegenden Erfindung ermöglicht, in einer Vertiefung, wie beispielsweise einer Kavität bzw. Kaverne in einem MECS-Sensorelement, nasschemische Elektroden einzusetzen und einen elektrischen Elektrodenkontakt mittels eines kompakten, trockenchemisch aufgebrachten Films aus der Kaverne heraus auf eine Rückseite des Sensors zu führen. Dabei kann insbesondere ein gerichtetes Abscheideverfahren eingesetzt werden, bei dem Abschattungseffekte bewirken können, dass beispielsweise lediglich Flanken einer Kaverne, nicht aber ein Kavernenboden beschichtet wird.

Es kann insbesondere eine Erschließung neuer Anwendungsfelder für die MEMS-Technologie (MEMS = mikro-elektromechanische Systeme) ermöglicht werden, wie beispielsweise durch die vorstehend genannte Kombination trockenchemisch und nasschemisch erzeugter Schichten für miniaturisierte Feststoffelektrolyt-Gassensoren bzw. MECS-Sensorelemente, die unter Einsatz mikromechanischer Verfahren und Prozesse gefertigt werden können. Solche Sensorelemente können beispielsweise zur Messung eines Restsauerstoffanteils bzw. zur sogenannten Lambda-Messung in Abgasen von Verbrennungsmotoren verwendet werden.

Ein mögliches Sensorkonzept für einen solchen Gassensor kann eine Verwendung einer freistehenden bzw. partiell freiliegenden Feststoffelektrolytmembran umfassen, wobei eine Freistellung in einer Variante durch Rückätzen eines Halbleitersubstrats bzw. Silizium-Wafers im Membranbereich erfolgen kann. Eine Tiefe einer so ausgeformten Kaverne kann dabei einer Dicke eines Wafers bzw. Substratmaterials entsprechen. Die freistehende Membran bzw. ein freiliegender Abschnitt der Feststoffelektrolytschicht wird auf beiden Seiten mit abgasresistenten und temperaturstabilen, porösen Elektroden bzw. Messelektroden beschichtet, wie beispielsweise aus Platin oder einem anderen Metall ausgeformt sein können. Eine Variante, um zum Beispiel funktionale, poröse Platin-Elektroden als Messelektroden erzeugen zu können, kann eine nasschemische Beschichtung von funktionalisierten Platin-Nanopartikeln aus einer flüssigen Lösung aufweisen. Durch Trocknen und Sintern von zunächst flüssigem Beschichtungsmaterial kann eine kompakte Schicht aus versinterten Platin-Partikeln erzeugt werden, die insbesondere resistent gegen Temperaturbeanspruchung und Feuchteeintrag ist.

Um verhindern zu können, dass es bei einer ganzflächigen nasschemischen Beschichtung an einem Halbleitersubstrat, beispielsweise auf einer Sensorrückseite, auf Kaverneninnenseiten und an Kanten zu einem Abplatzen der Beschichtung und zu Filmrissen kommt, kann gemäß Ausführungsformen der vorliegenden Erfindung für eine durchgehende, elektrisch leitfähige Kontaktierung von einer Elektrolytrückseite auf eine Waferrückseite insbesondere auf eine nasschemische Aufbringung durchgehender bzw. ganzflächiger Elektrodenfilme verzichtet werden. Daher können zur Signalleitung bzw. zum elektrischen Anschluss der Messelektroden trockenchemisch aufgebrachte Elektroden verwendet werden, da nasschemisch aufgebrachte Elektroden hierfür ohnehin eine geringere spezifische Leitfähigkeit als beispielsweise gesputterte, relativ porenfreie Metallschichten aufweisen, was eine Übertragung eines Spannungssignals über eine längere Strecke, wie beispielsweise von der Feststoffelektrolytschicht bis zu einem Sensor-Steckerkontakt oder dergleichen erschweren würde. Abhilfe kann hier durch eine Kombination aus einem trockenchemischen Abscheideverfahren, wie beispielsweise Sputtern oder Elektronenstrahlverdampfen, auf einer von der Feststoffelektrolytschicht abgewandten Seite des Halbleitersubstrats bzw. einer Waferrückseite und Kavernenwänden und einer nasschemischen Abscheidung einer porösen Messelektrodenschicht, beispielsweise aus Platin, auf einer Elektrolytoberfläche am Kavernenboden geschaffen werden. Dazu kann für eine Realisierung einer solchen Kombination zweier Beschichtungsverfahren eine trockenchemisch abgeschiedene, beispielsweise gesputterte, geschlossene und gasdichte Signalleiterschicht aus Platin oder dergleichen auf Kavernenwände und Waferrückseite beschränkt angeordnet werden.

Während bei einer Standard-MEMS-Fertigung normalerweise zur Erfüllung einer solchen Anforderung Lithografieprozesse eingesetzt werden können, in denen ein ganzflächig aufgebrachter fotosensitiver Lack über Lithografiemasken lokal strukturiert werden kann und anschließend eine Strukturübertragung durch Ätzprozesse oder einen Lift-Off-Prozess erfolgen kann, kann gemäß Ausführungsformen der vorliegenden Erfindung insbesondere eine lokale Strukturierung auch im Falle extremer Topografien erreicht werden bzw. kann eine lokal begrenzte Schichtabscheidung bzw. Strukturdefinition auch auf extrem geformten Oberflächentopografien ermöglicht werden. Somit kann die Kombination trockenchemisch und nasschemisch erzeugter Schichten bzw. Filme auch auf extremen Oberflächentopografien, wie zum Beispiel bei Vorliegen tiefer Kavernen, realisiert werden, was beispielsweise mittels Fotolithografie aufwendiger bis unmöglich oder unzufriedenstellend wäre. Anders als bei der Fotolithografie kann somit insbesondere auch an stark topografisch geformten Oberflächen gemäß Ausführungsformen der vorliegenden Erfindung verhindert werden, dass ein Strukturverlust an tiefen Stellen aufgrund diffuser Lichtstreuung durch einen erhöhten Abstand zwischen Lithografiemaske und Fotolack, ein Lackabriss an Strukturkanten beim Belacken einer Waferoberfläche oder dergleichen auftritt.

Es wird ein Verfahren zum Herstellen einer Gassensorvorrichtung zum Erfassen zumindest eines gasförmigen Analyten vorgestellt, wobei das Verfahren die gemäß dem Hauptanspruch definierten Schritte aufweist.

Unter einem ausgesparten Abschnitt einer Schicht kann vorliegend beispielsweise eine Öffnung in der betreffenden Schicht verstanden werden. Hierbei kann die Öffnung auch teilweise wieder verfüllt sein, jedoch durch Material der betreffenden Schicht vollständig umschlossen sein.

Die Gassensorvorrichtung kann ausgebildet sein, um den zumindest einen gasförmigen Analyten qualitativ und zusätzlich oder alternativ quantitativ zu erfassen. Der zumindest eine gasförmige Analyt kann beispielsweise Sauerstoff und zusätzlich oder alternativ Stickoxide umfassen. Dabei kann die Gassensorvorrichtung beispielsweise für eine Abgassensorik in Kraftfahrzeugen, für eine Abgassensorik in beispielsweise stationären Heizanlagen, für Brandmeldesysteme, für eine Gassensorik in mobilen elektronischen Geräten, wie beispielsweise Mobiltelefonen, für eine Atemgasanalyse, für eine industrielle Prozessüberwachung etc. eingesetzt werden. Insbesondere kann die Gassensorvorrichtung beispielsweise in Kraftfahrzeugen eingesetzt werden und ausgebildet sein, um Abgasnachbehandlungssysteme zu überwachen bzw. zu regeln. Das Halbleitersubstrat kann aus zumindest einem von mehreren Materialien ausgeformt sein, die mittels Prozessen aus dem Bereich der Halbleiterbearbeitung bearbeitet werden können. Die Festelektrolytschicht kann ein Ionen leitendes, beispielsweise keramisches Material aufweisen, insbesondere ein Sauerstoffionen leitendes Keramikmaterial.

Gemäß dem Hauptanspruch wird im Schritt des Erzeugens die Signalleiterschicht trockenchemisch abgeschieden und mittels selektiver Laserablation in dem zumindest einen Aussparungsabschnitt entfernt.

Hierbei können die Signalleiterschicht und die Festelektrolytschicht unterschiedliche optische Eigenschaften und zusätzlich oder alternativ mechanische Eigenschaften aufweisen, wie beispielsweise optische Absorption, Festigkeit, Wärmeausdehnung oder dergleichen. Bei einem Ausführen der Laserablation kann ein Laserstrahlprofil adaptiv an eine Strukturhöhe des Halbleitersubstrats und des zumindest einen Kavitätsabschnitts angepasst werden. Eine solche Ausführungsform bietet den Vorteil, dass der zumindest eine Aussparungsabschnitt in der Signalleiterschicht auf präzise Weise ausgeformt werden kann. Zudem kann selbst über starke Oberflächentopografien hinweg ein gleichmäßiger Grad einer Ablation bzw. Entschichtung unabhängig von einer Strukturhöhe erzielt werden.

Dabei wird die selektive Laserablation von der Halbleitersubstratseite des Sensorgrundkörpers her ausgeführt werden. Alternativ kann die Laserablation von der Festelektrolytschichtseite des Sensorgrundkörpers der durch die Festelektrolytschicht hindurch ausgeführt werden. Hierbei ist die Festelektrolytschicht für bei der Laserablation verwendetes Laserlicht transparent. Eine solche Ausführungsform bietet den Vorteil, dass eine Flexibilität bei einer Prozessgestaltung bzw. Verfahrensgestaltung erhöht und somit gegebenenfalls eine Herstellung der Sensorvorrichtung erleichtert werden kann.

Gemäß einer Ausführungsform kann im Schritt des Erzeugens die Signalleiterschicht in einer definierten Abscheiderichtung trockenchemisch abgeschieden werden. Dabei kann in Abhängigkeit von der Abscheiderichtung und einer Geometrie des zumindest einen Kavitätsabschnitts der zumindest eine Aussparungsabschnitt vor einem Material der Signalleiterschicht abgeschattet werden. Hierbei kann zumindest die in dem Kavitätsabschnitt des Halbleitersubstrats freiliegende Festelektrolytschicht in Abhängigkeit von der Abscheiderichtung relativ zu einer Bezugsebene des Sensorgrundkörpers bzw. in Abhängigkeit von einer Ausrichtung des Sensorgrundkörpers bezüglich einer Abscheidequelle in einem Strömungsschatten des gerichtet abgeschiedenen Materials der Signalleiterschicht angeordnet sein. Eine solche Ausführungsform bietet den Vorteil, dass die Signalleiterschicht mit dem zumindest einen Aussparungsabschnitt mittels eines besonders einfachen Prozesses in beispielsweise lediglich einem Schritt erzeugt werden kann.

Auch kann im Schritt des Bereitstellens ein Sensorgrundkörper bereitgestellt werden, in dessen Halbleitersubstrat der zumindest eine Kavitätsabschnitt mit einer Tiefe von mehr als 100 Mikrometern oder mehr als 200 Mikrometern ausgeformt ist.

Ferner kann das Verfahren einen Schritt des Beschichtens des Halbleitersubstrats mit einer Passivierungsschicht ausweisen. Dabei kann der Schritt des Beschichtens vor dem Schritt des Erzeugens der Signalleiterschicht durchgeführt werden. Somit kann die Signalleiterschicht zumindest teilflächig auf der Passivierungsschicht erzeugt werden. Eine solche Ausführungsform bietet den Vorteil, dass eine Degradation einer Oberfläche des Halbleitersubstrats bei einem Betrieb der Sensorvorrichtung, insbesondere bei Einsatz im Abgas von Verbrennungsmotoren, vermieden werden kann.

Zudem kann im Schritt des Erzeugens die Signalleiterschicht trockenchemisch abgeschieden und mit einer Maskierungsschicht abgedeckt werden. Hierbei kann die Maskierungsschicht mittels selektiver Laserablation lokal in mindestens einem Aussparungsabschnitt abgetragen werden und die Signalleiterschicht in diesem oder falls benötigt in mehreren der erzeugten Aussparungsabschnitte entfernt werden. Auch kann die Maskierungsschicht beseitigt werden. Somit kann der Schritt des Erzeugens einen Teilschritt des Abscheidens, einen Teilschritt des Abdeckens, einen Teilschritt des Abtragens und einen Teilschritt des Entfernens sowie einen optionalen Teilschritt des Beseitigens aufweisen. Dabei können die Teilschritte in vorstehend genannter Reihenfolge ausgeführt werden. Eine solche Ausführungsform bietet den Vorteil, dass die Signalleiterschicht mit dem zumindest einen Aussparungsabschnitt auch in Fällen erzeugt werden kann, in denen ein direktes Ablatieren bzw. Auferieren der Signalleiterschicht schwierige oder unmöglich wäre.

Es wird ferner beispielhaft eine Gassensorvorrichtung zum Erfassen zumindest eines gasförmigen Analyten vorgestellt, die nicht unter den Schutzumfang der Ansprüche fällt, wobei die Gassensorvorrichtung folgende Merkmale aufweist:
einen Sensorgrundkörper, der ein Halbleitersubstrat, in dem zumindest ein Kavitätsabschnitt ausgeformt ist, und eine an einer ersten Hauptoberfläche des Halbleitersubstrats angeordnete Festelektrolytschicht aufweist, wobei die Festelektrolytschicht in dem zumindest einen Kavitätsabschnitt durch das Halbleitersubstrat ausgespart ist;
eine Signalleiterschicht, die an einer Halbleitersubstratseite des Sensorgrundkörpers trockenchemisch abgeschieden ist, wobei im Bereich der in dem zumindest einen Kavitätsabschnitt durch das Halbleitersubstrat unbedeckten Festelektrolytschicht zumindest ein Aussparungsabschnitt in der Signalleiterschicht ausgeformt ist, in dem die Signalleiterschicht ausgespart ist; und
zumindest zwei Messelektroden, die mittels eines nasschemischen Prozesses auf die Festelektrolytschicht aufgebracht sind, wobei eine erste Messelektrode in dem zumindest einen Aussparungsabschnitt der Signalleiterschicht angeordnet ist und eine zweite Messelektrode auf einer Festelektrolytschichtseite des Sensorgrundkörpers angeordnet ist.

Die Gassensorvorrichtung kann durch Ausführen einer Ausführungsform des vorstehend genannten Verfahrens hergestellt sein oder werden.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen, wobei eine solche Vorrichtung nicht unter den Schutzumfang der Ansprüche fällt.

Unter einer beispielhaften Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Beispielhaft ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Die beispielhaft vorstehend genannte Gassensorvorrichtung kann in Mikrosystemtechnik als Plattform bzw. Grundbaustein verwendet werden, um weitere Sensorfunktionen, wie beispielsweise Druckerfassung, Partikelerfassung, Lambda-Sensorik, Kohlenwasserstofferfassung etc. auf einem einzigen Chip zu integrieren und kombiniert auszuwerten.

Der hier vorgestellte Ansatz wird nachstehend anhand der beigefügten Zeichnungen beispielhaft näher erläutert. Es zeigen:
Fig. 1 eine schematische Schnittdarstellung einer Gassensorvorrichtung hergestellt gemäß einem Ausführungsbeispiel der vorliegenden Erfindung;
Figuren 2A bis 6B schematische Schnittdarstellungen von Gassensorvorrichtungen gemäß Ausführungsbeispielen der vorliegenden Erfindung in verschiedenen Herstellungszuständen; und
Fig. 7 ein Ablaufdiagramm eines Verfahrens gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

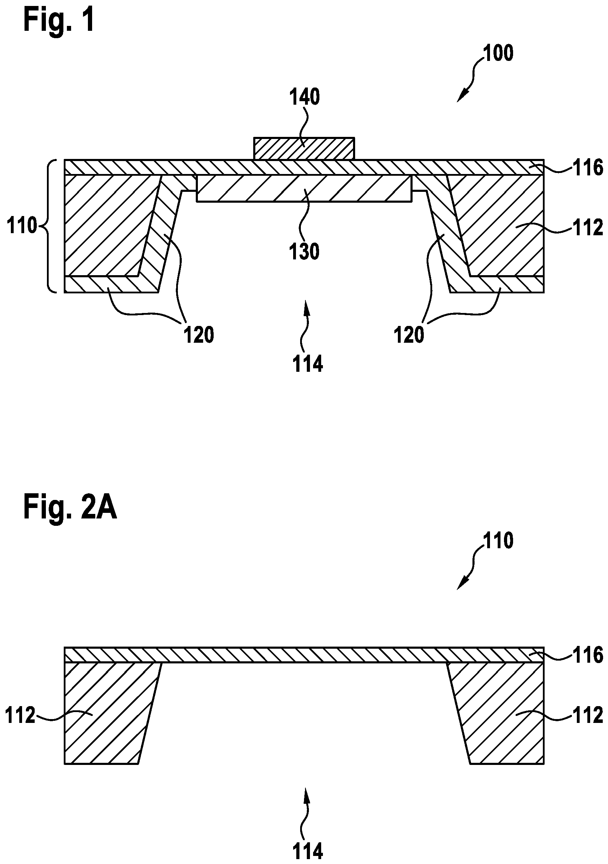

Fig. 1 zeigt eine schematische Schnittdarstellung einer Gassensorvorrichtung 100 hergestellt gemäß einem Ausführungsbeispiel der vorliegenden Erfindung. Die Gassensorvorrichtung 100 ist zum Erfassen zumindest eines gasförmigen Analyten ausgebildet. Bei dem zumindest einen gasförmigen Analyten handelt es sich beispielsweise hierbei um Sauerstoff. Die Gassensorvorrichtung 100 ist als ein mikroelektrochemischer Sensor bzw. Gassensor, insbesondere Sauerstoffsensor ausgeführt. Dabei ist die Gassensorvorrichtung 100 beispielsweise für eine Gassensorik bzw. Abgassensorik in Kraftfahrzeugen, stationären Heizanlagen, Brandmeldesystemen, mobilen elektronischen Geräten, wie beispielsweise Mobiltelefonen, Atemgasanalysegeräten, für eine industrielle Prozessüberwachung etc. einsetzbar.

Die Gassensorvorrichtung 100 weist einen Sensorgrundkörper 110 auf. Der Sensorgrundkörper 110 umfasst ein Halbleitersubstrat 112. Das Halbleitersubstrat 112 weist beispielsweise Silizium als Substratmaterial auf. Auch weist das Halbleitersubstrat 112 eine erste Hauptoberfläche und eine gegenüberliegende, zweite Hauptoberfläche auf. In dem Halbleitersubstrat 112 ist gemäß dem in Fig. 1 dargestellten Beispiel beispielhaft lediglich ein Kavitätsabschnitt 114 bzw. Ausnehmungsabschnitt ausgeformt. Hierbei erstreckt sich der Kavitätsabschnitt 114 als eine Durchgangsöffnung durch das Halbleitersubstrat 112 in durch von der zweiten Hauptoberfläche bis zu der ersten Hauptoberfläche des Halbleitersubstrats 112. Der Kavitätsabschnitt 114 weist beispielsweise ein trapezförmiges Schnittprofil auf. Dabei verjüngt sich das Schnittprofil des Kavitätsabschnitts 114 von der zweiten Hauptoberfläche zu der ersten Hauptoberfläche des Halbleitersubstrats 112 hin. Der Sensorgrundkörper 110 umfasst ferner eine Festelektrolytschicht 116. Die Festelektrolytschicht 116 ist an der ersten Hauptoberfläche des Halbleitersubstrats 112 angeordnet. In dem Kavitätsabschnitt 114 ist die Festelektrolytschicht 116 durch das Halbleitersubstrat 112 unbedeckt. Somit umfasst der Sensorgrundkörper 110 das Halbleitersubstrat 112 mit dem Kavitätsabschnitt 114 und die Festelektrolytschicht 116. Der Sensorgrundkörper 110 weist daher eine Festelektrolytschichtseite und eine Halbleitersubstratseite auf.

Die Gassensorvorrichtung 100 weist auch eine Signalleiterschicht 120 auf. Die Signalleiterschicht 120 ist an der Halbleitersubstratseite des Sensorgrundkörpers 110 mittels trockenchemischer Abscheidung aufgebracht. Die Signalleiterschicht 120 ist aus einem elektrisch leitfähigen Material ausgeformt. Gemäß dem in Fig. 1 dargestellten Beispiel bedeckt die Signalleiterschicht 120 auf der Halbleitersubstratseite des Sensorgrundkörpers 110 eine Oberfläche des Halbleitersubstrats 112 und einen Teilabschnitt der Festelektrolytschicht 116 in dem Kavitätsabschnitt 114. Anders ausgedrückt bedeckt die Signalleiterschicht 120 hierbei die zweite Hauptoberfläche des Halbleitersubstrats 112, Flanken des Kavitätsabschnitts 114 und den Teilabschnitt der Festelektrolytschicht 116 in dem Kavitätsabschnitt 114. Die Signalleiterschicht 120 weist im Bereich der in dem Kavitätsabschnitt 114 durch das Halbleitersubstrat 112 unbedeckten Festelektrolytschicht 116 einen Aussparungsabschnitt auf, in dem die Signalleiterschicht 120 entfernt oder unabgeschieden (also ausgespart) ist.

Die Gassensorvorrichtung 100 weist gemäß dem in Fig. 1 dargestellten Beispiel ferner beispielhaft lediglich zwei Messelektroden 130 und 140 auf. Die Messelektroden 130 und 140 sind an der Festelektrolytschicht 116 aufgebracht. Hierbei sind die Messelektroden 130 und 140 mittels eines nasschemischen Prozesses auf die Festelektrolytschicht 116 aufgebracht. Eine erste Messelektrode 130 ist in dem Aussparungsabschnitt der Signalleiterschicht 120 angeordnet. Eine zweite Messelektrode 140 ist auf einer Festelektrolytschichtseite des Sensorgrundkörpers 110 angeordnet. Die Messelektrode 130 und 140 sind für eine elektrochemische Wechselwirkung mit dem zumindest einen gasförmigen Analyten ausgebildet. In dem Aussparungsabschnitt ist die Signalleiterschicht 120 durch die erste Messelektrode 130 unterbrochen. Die erste Messelektrode 130 ist mittels der Signalleiterschicht 120 elektrisch leitfähig ankontaktiert. In der Schnittdarstellung von Fig. 1 weist die erste Messelektrode 130 hierbei zwei Grenzflächen mit der Signalleiterschicht 120 auf.

Gemäß einem Beispiel kann die Signalleiterschicht 120 einen geringeren Anteil des Halbleitersubstrats 112 und/oder der Festelektrolytschicht 116 in dem Kavitätsabschnitt 114 bedecken und optional eine kleinere Grenzfläche mit der ersten Messelektrode 130 aufweisen als bei dem in Fig. 1 dargestellten Ausführungsbeispiel.

Figuren 2A bis 2G zeigen schematische Schnittdarstellungen von mindestens Teilabschnitten einer nach einem Ausführungsbeispiel der vorliegenden Erfindung hergestellten Gassensorvorrichtung in verschiedenen Herstellungszuständen bei Ausführung einer Variante eines Herstellungsverfahrens. Bei der Gassensorvorrichtung handelt es sich beispielsweise um die Gassensorvorrichtung aus Fig. 1.

Fig. 2A zeigt hierbei eine schematische Schnittdarstellung eines Sensorgrundkörpers 110 der Gassensorvorrichtung. Dabei ist der Sensorgrundkörper 100 nach einem Schritt des Bereitstellens des Sensorgrundkörpers 110 des Herstellungsverfahrens gezeigt. Der Sensorgrundkörper 110 weist das Halbleitersubstrat 112 mit dem Kavitätsabschnitt 114 und die Festelektrolytschicht 116 auf. Die Festelektrolytschicht 116 ist beispielsweise als eine partiell freiliegende bzw. freistehende Yttrium-stabilisierte Zirconoxid-Elektrolytmembran bzw. YSZ-Elektrolytmembran ausgeführt.

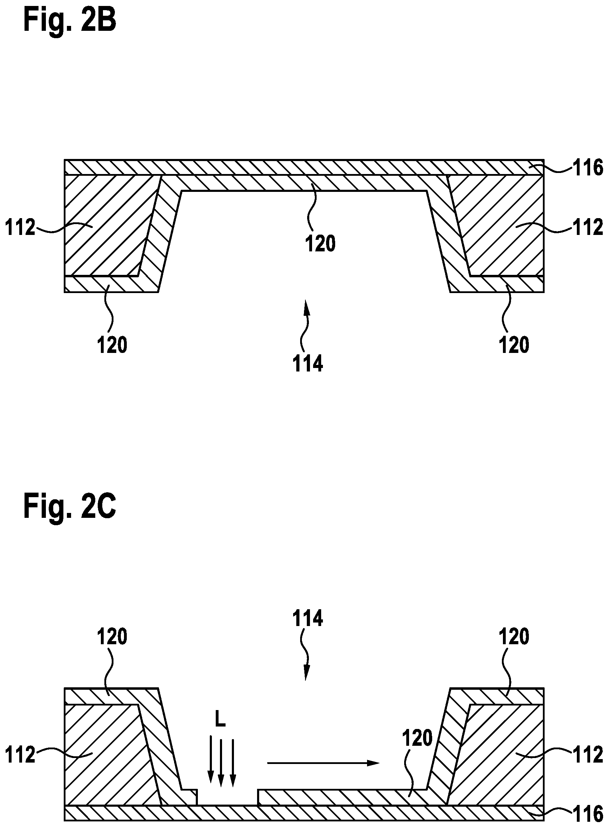

Fig. 2B zeigt eine schematische Schnittdarstellung des Sensorgrundkörpers aus Fig. 2A mit abgeschiedener Signalleiterschicht 120. Somit ist hierbei der Sensorgrundkörper in einem Herstellungszustand gezeigt, in dem die Signalleiterschicht 120 mittels trockenchemischer Abscheidung, insbesondere mittels trockenchemischer Platin-Beschichtung, an der Halbleitersubstratseite des Sensorgrundkörpers erzeugt ist. Hierbei ist die Signalleiterschicht 120 ganzflächig an der Halbleitersubstratseite des Sensorgrundkörpers abgeschieden.

Fig. 2C zeigt eine schematische Schnittdarstellung des Sensorgrundkörpers mit der Signalleiterschicht 120 aus Fig. 2B bei Ablauf einer Laserablation im Rahmen des Herstellungsverfahrens. Mittels eines Lasers L wird ein an die Festelektrolytschicht 116 angrenzender Teilabschnitt der Signalleiterschicht 120 in dem Kavitätsabschnitt 114 durch Laserablation entfernt. Hierbei ist der Laser L auf Seiten des Kavitätsabschnitts 114 angeordnet. Es erfolgt somit eine lokale Laserablation in dem Kavitätsabschnitt 114 bzw. am Kavernenboden. In Fig. 2C ist eine Bewegungsrichtung des Lasers L symbolisch durch einen Pfeil veranschaulicht.

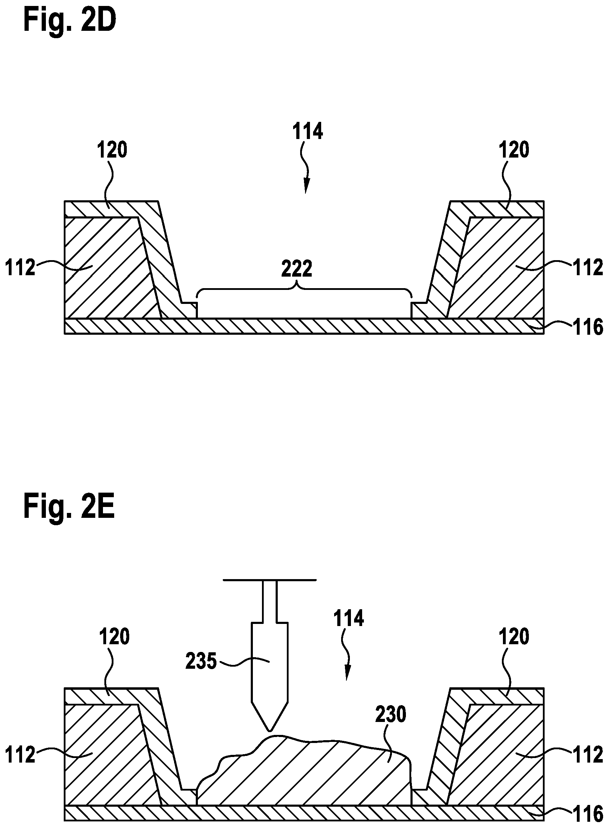

Fig. 2D zeigt eine schematische Schnittdarstellung des Sensorgrundkörpers mit der Signalleiterschicht 120 aus Fig. 2C nach der Laserablation. Hierbei ist der an die Festelektrolytschicht 116 angrenzende Teilabschnitt der Signalleiterschicht 120 in dem Kavitätsabschnitt 114 durch Laserablation entfernt. Somit ist in der Signalleiterschicht 120 der in Fig. 1 erwähnte Aussparungsabschnitt 222 erzeugt. In dem Aussparungsabschnitt 222 ist ein Material der Signalleiterschicht 120 entfernt. Anders ausgedrückt ist die Signalleiterschicht 120 in dem Aussparungsabschnitt 222 unterbrochen. Somit ist die Gassensorvorrichtung in Fig. 2D in einem teilgefertigten Zustand gezeigt.

Bei einer solchen selektiven Ablation von Platin auf YSZ mittels Laser L wird das Platin der Signalleiterschicht 120 in dem Aussparungsabschnitt 222 beispielsweise lokal durch einzelne Laserpulse entfernt. Insbesondere durch Platzieren der Laserpulse direkt nebeneinander mit einem gewissen Überlapp ist eine flächige Ablation realisierbar. Um während der Ablation einen Laserfokus auf eine sich veränderte Strukturhöhe anpassbar zu machen, ist eine Höheninformation entweder in einem Laserablationsprogramm, welches in einer Laservorrichtung programmierbar ist, hinterlegbar oder ist ein adaptives Fokussystem einsetzbar, das vor Aussendung von Ablationspulsen über ein Autofokussystem den Laserfokus an die tatsächliche Strukturhöhe anpasst. Ebenfalls ist eine Strahlformung mit Hilfe eines Axikons realisierbar, wobei sowohl eine hohe Tiefenschärfe für einen robusten Prozess als auch für eine Herstellung kleiner Strukturgrößen erreichbar ist.

Fig. 2E zeigt eine schematische Schnittdarstellung der teilgefertigten Gassensorvorrichtung aus Fig. 2D mit einem aufgebrachten Elektrodenmaterial 230 für eine Messelektrode. Dabei ist das Elektrodenmaterial 230 in dem Aussparungsabschnitt auf die Festelektrolytschicht 116 aufgebracht. Genau gesagt ist das Elektrodenmaterial 230 in dem Aussparungsabschnitt der Signalleiterschicht 120 nasschemisch auf die Festelektrolytschicht 116 aufgebracht. Bei dem Elektrodenmaterial 230 handelt es sich beispielsweise um ein platinhaltiges Material, insbesondere eine Platin-Nanopartikellösung. Das nasschemische Aufbringen des Elektrodenmaterials 230 erfolgt hierbei zum Beispiel durch Dispensen mittels eines Aufbringwerkzeugs 235.

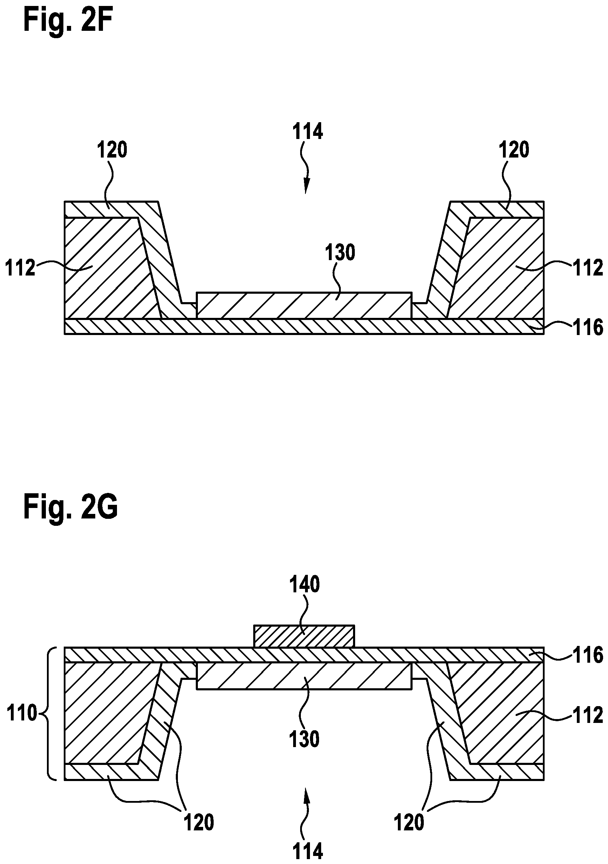

Fig. 2F zeigt eine schematische Schnittdarstellung der teilgefertigten Gassensorvorrichtung aus Fig. 2E mit gesintertem Elektrodenmaterial bzw. hergestellter erster Messelektrode 130. Somit ist in Fig. 2F die teilgefertigte Gassensorvorrichtung in einem Zustand dargestellt, in dem das Elektrodenmaterial aus Fig. 2E durch Sintern verfestigt ist und somit die erste Messelektrode 130 erzeugt ist. Die erste Messelektrode 130 ist in dem Aussparungsabschnitt der Signalleiterschicht 120 an der Festelektrolytschicht 116 angeordnet.

Fig. 2G zeigt eine schematische Schnittdarstellung der Gassensorvorrichtung 100 mit erzeugter bzw. aufgebrachter zweiter Messelektrode 140 bzw. Vorderseitenelektrode. Somit ist die Gassensorvorrichtung 100 in Fig. 2G in einem Zustand gezeigt, der dem in Fig. 1 dargestellten Zustand entspricht. Beispielsweise handelt es sich bei dem in Fig. 2G gezeigten Zustand der Gassensorvorrichtung 100 um einen hergestellten bzw. gefertigten Zustand.

Die Figuren 2A bis 2G zeigen Herstellungszustände der Gassensorvorrichtung 100 bzw. des MECS-Sensorelements bei Ablauf eines Herstellungsverfahrens unter Einsatz der Laserablation, um eine Verwendung funktionaler, nasschemisch aufgebrachter Elektroden für eine Rückseitenkontaktierung der Festelektrolytschicht 116 bzw. einer Elektrolytmembran zu ermöglichen. Anders ausgedrückt zeigen die Figuren 2A bis 2G eine Anwendung der Laserablation für ein Herstellungsverfahren der Gassensorvorrichtung 100 mit kombinierter nasschemischer und trockenchemischer Abscheidung. Hierbei erfolgt eine selektive Laserablation der Signalleiterschicht 120, beispielsweise einer gesputterten Platin-Schicht, in dem Aussparungsabschnitt 222 von der angrenzenden Festelektrolytschicht 116 in dem Kavitätsabschnitt 114, der insbesondere eine über 200 Mikrometer tiefe Kavität aufweist.

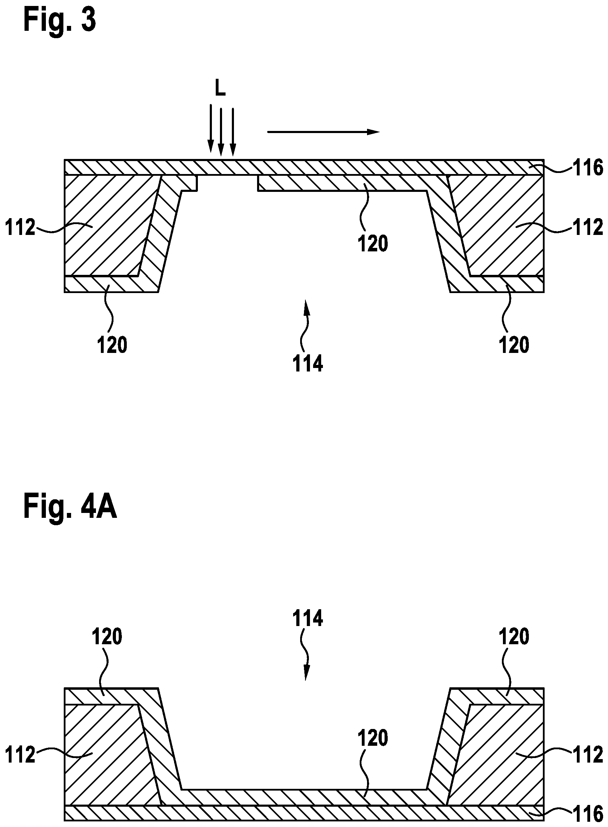

Fig. 3 zeigt eine schematische Schnittdarstellung eines Sensorgrundkörpers einer Gassensorvorrichtung bei Ablauf einer Laserablation im Rahmen eines Herstellungsverfahrens. Bei dem Sensorgrundkörper handelt es sich beispielsweise um einen Sensorgrundkörper wie den Sensorgrundkörper aus Fig. 2C bzw. es entspricht die Darstellung in Fig. 3 der Darstellung aus Fig. 2C mit der Ausnahme, dass die Festelektrolytschicht 116 für einen Laser L Transparent ausgeformt ist. Somit wird auch in Fig. 3 mittels des Lasers L der an die Festelektrolytschicht 116 angrenzende Teilabschnitt der Signalleiterschicht 120 in dem Kavitätsabschnitt 114 durch Laserablation entfernt. Hierbei ist der Laser L jedoch auf Seiten der Festelektrolytschicht 116 angeordnet. Es erfolgt eine lokale Laserablation in dem Kavitätsabschnitt 114 bzw. am Kavernenboden. Auch in Fig. 3 ist eine Bewegungsrichtung des Lasers L symbolisch durch einen Pfeil veranschaulicht. Hierbei erfolgt die Laserablation durch die transparent ausgeführte Festelektrolytschicht 116 hindurch auf den zu entfernenden Teilabschnitt der Signalleiterschicht 120.

Hierbei ist die Signalleiterschicht 120 als zu ablatierende Schicht auf die als optisch transparente Membran ausgeführte Festelektrolytschicht 116 abgeschieden. Somit ist eine Lasereinwirkung von Seiten der Festelektrolytschicht 116 her bzw. von der Wafervorderseite möglich, um die auf der Membranrückseite abgeschiedene Signalleiterschicht 120 mit dem Laser L zu entfernen. In diesem Fall erfolgt ein maximaler Energieeintrag des Lasers L an einer Grenzfläche zwischen Festelektrolytschicht 116 und Signalleiterschicht 120, wobei zunächst direkt an die Festelektrolytschicht 116 angrenzende Atomlagen verdampfen. Somit erfolgt ein sauberer Schichtabtrag von der Festelektrolytschicht-Signalleiterschicht-Grenzfläche aus. Hierdurch ist eine notwendige Pulsenergie des Lasers L reduzierbar, sodass sowohl schnelle Prozesszeiten als auch eine schädigungsarme Ablation realisierbar ist. Zum Erzeugen des Lasers L sind hierbei gepulste Strahlquellen einsetzbar, insbesondere mit Pulslängen im Bereich einiger Picosekunden und kürzer bzw. kurzen Wellenlängen, beispielsweise frequenzverdoppelte und -verdreifachte YAG-Festkörperlaser (YAG = Yttrium-Aluminium-Granat).

Figuren 4A bis 4F zeigen schematische Schnittdarstellungen von mindestens Teilabschnitten einer Gassensorvorrichtung gemäß einem Ausführungsbeispiel der vorliegenden Erfindung in verschiedenen Herstellungszuständen bei Ausführung einer Variante eines Herstellungsverfahrens. Bei der Gassensorvorrichtung handelt es sich beispielsweise um die Gassensorvorrichtung aus Fig. 1.

Fig. 4A zeigt eine schematische Schnittdarstellung eines Sensorgrundkörpers der Gassensorvorrichtung mit abgeschiedener Signalleiterschicht 120. Somit ist hierbei der Sensorgrundkörper in einem Herstellungszustand gezeigt, in dem die Signalleiterschicht 120 mittels trockenchemischer Abscheidung, insbesondere mittels trockenchemischer Platin-Beschichtung, an der Halbleitersubstratseite des Sensorgrundkörpers erzeugt ist. Hierbei ist die Signalleiterschicht 120 ganzflächig an der Halbleitersubstratseite des Sensorgrundkörpers abgeschieden.

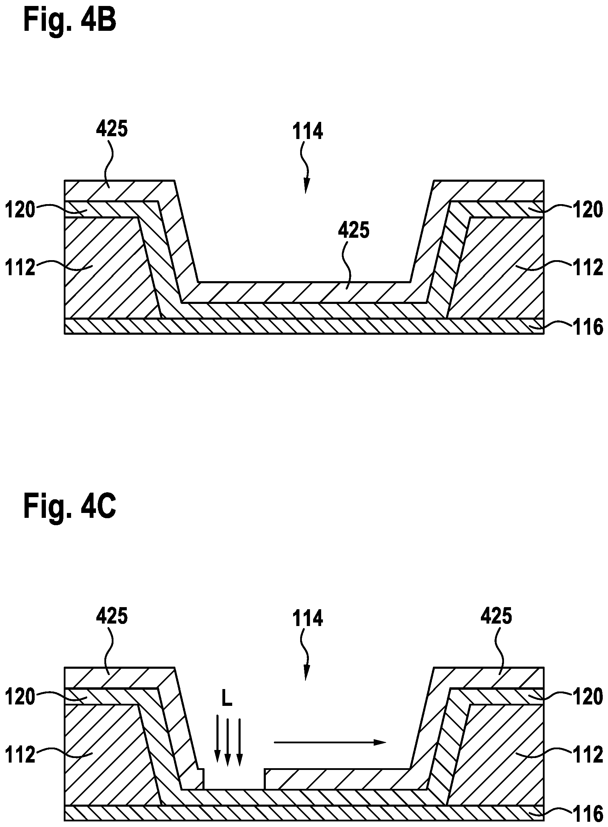

Fig. 4B zeigt eine schematische Schnittdarstellung des Sensorgrundkörpers der Gassensorvorrichtung mit abgeschiedener Signalleiterschicht 120 aus Fig. 4A mit zusätzlich aufgebrachter Maskierungsschicht 425. Hierbei ist auf der abgeschiedenen Signalleiterschicht 120 eine Maskierungsschicht 425 aufgebracht.

Fig. 4C zeigt eine schematische Schnittdarstellung des Sensorgrundkörpers der Gassensorvorrichtung mit abgeschiedener Signalleiterschicht 120 und aufgebrachter Maskierungsschicht 425 aus Fig. 4B bei Ablauf einer Laserablation. Mittels eines Lasers L wird ein an den in der Signalleiterschicht 120 auszuformenden Aussparungsabschnitt angrenzender Teilabschnitt der Maskierungsschicht 425 in dem Kavitätsabschnitt 114 durch Laserablation entfernt. Hierbei ist der Laser L auf Seiten des Kavitätsabschnitts 114 angeordnet. In Fig. 4C ist eine Bewegungsrichtung des Lasers L symbolisch durch einen Pfeil veranschaulicht.

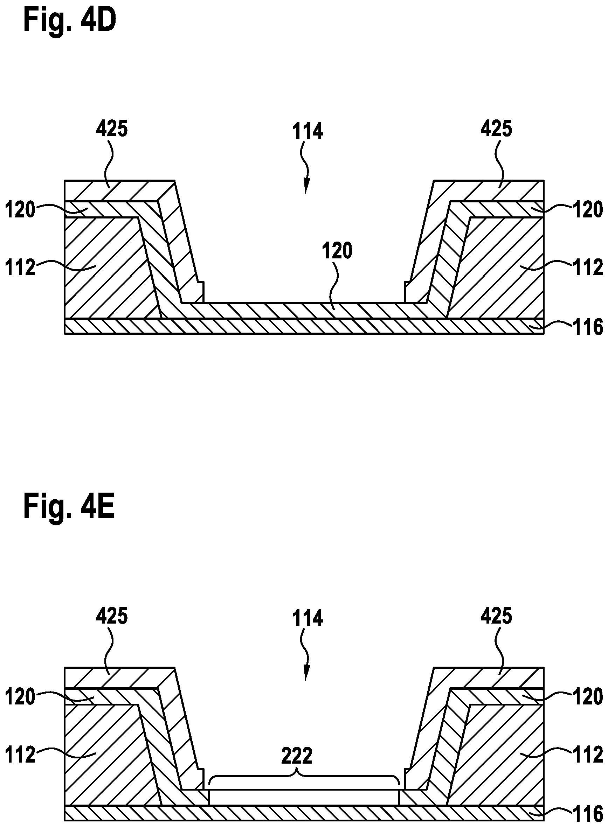

Fig. 4D zeigt eine schematische Schnittdarstellung des Sensorgrundkörpers mit der Signalleiterschicht 120 und der Maskierungsschicht 425 aus Fig. 4C nach der Laserablation. Hierbei ist der an den Aussparungsabschnitt angrenzende Teilabschnitt der Maskierungsschicht für 125 in dem Kavitätsabschnitt 114 durch Laserablation entfernt. Somit liegt ein Teilabschnitt der Signalleiterschicht frei, in dem der Aussparungsabschnitt auszuformen ist. Anders ausgedrückt zeigt Fig. 4D den Sensorgrundkörper aus Fig. 4C nach einer selektiven Laserablation eines Teilabschnittes der Maskierungsschicht 425.

Fig. 4E zeigt eine schematische Schnittdarstellung des Sensorgrundkörpers mit der durch Laserablation partiell entfernten Maskierungsschicht 425 aus Fig. 4D nach einem partiellen Entfernen der Signalleiterschicht 120 in einem Aussparungsabschnitt 222. Hierbei ist in dem Aussparungsabschnitt 222 ein an die Festelektrolytschicht 116 angrenzender Teilabschnitt der Signalleiterschicht 120 in dem Kavitätsabschnitt 114 beispielsweise durch reaktives lonenätzen oder lonenstrahlätzen entfernt. Somit ist in der Signalleiterschicht 120 der in Fig. 1 erwähnte Aussparungsabschnitt 222 erzeugt. In dem Aussparungsabschnitt 222 ist ein Material der Signalleiterschicht 120 entfernt. Anders ausgedrückt ist die Signalleiterschicht 120 in dem Aussparungsabschnitt 222 unterbrochen. Somit ist die Gassensorvorrichtung in Fig. 4E in einem teilgefertigten Zustand nach Strukturübertragung der Maskierungsschicht 425 auf die Signalleiterschicht 120 gezeigt.

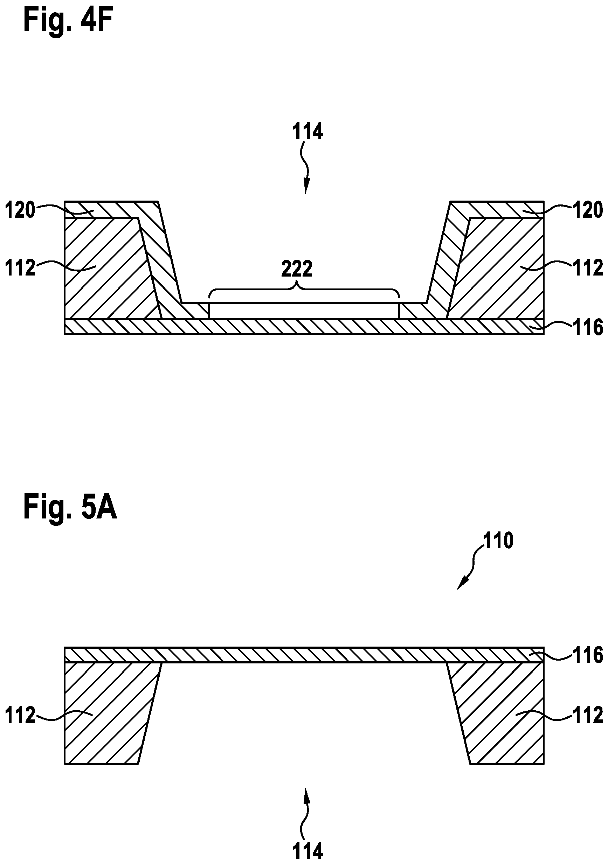

Fig. 4F zeigt eine schematische Schnittdarstellung der teilgefertigten Gassensorvorrichtung aus Fig. 2E nach einem Entfernen der Maskierungsschicht. Somit sind in Fig. 4F von der Gassensorvorrichtung hierbei das Halbleitersubstrat 112 mit dem Kavitätsabschnitt 114, die Festelektrolytschicht 116 und die Signalleiterschicht 120 mit dem Aussparungsabschnitt 222 dargestellt. Die Maskierungsschicht ist dabei insbesondere trockenchemisch oder nasschemisch entfernt worden.

Anders ausgedrückt ist in den Figuren 4A bis 4F eine Ablation unter Einsatz der Maskierungsschicht 420 dargestellt. In dem entsprechenden Herstellungsverfahren wird dabei zunächst auf die zu strukturierende Signalleiterschicht 120 die Maskierungsschicht 425 aufgebracht, wie es aus einer vergleichenden Betrachtung von Fig. 4A und Fig. 4B ersichtlich ist, wird die Maskierungsschicht 425 dann partiell ablatiert, wie es aus Fig. 4C und Fig. 4D ersichtlich ist, und wird eine Struktur der Maskierungsschicht 425 dann in die zu Signalleiterschicht 120 übertragen, wie es aus einer vergleichenden Betrachtung von Fig. 4D und Fig. 4E ersichtlich ist. Falls notwendig, wird die Maskierungsschicht 424 danach entfernt, wie es aus einer vergleichenden Betrachtung von Fig. 4E und Fig. 4F ersichtlich ist.

Falls direktes Ablatieren der Signalleiterschicht 120 nicht möglich sein sollte, besteht somit die Möglichkeit, auf die Signalleiterschicht 120 zunächst eine Maskierungsschicht 425 durch trockenchemische Abscheidung aufzubringen und diese Maskierungsschicht 425 dann zu ablatieren. Anschließend erfolgt die Strukturübertragung auf die Signalleiterschicht 120 und dann, falls notwendig, eine Entfernung der Maskierungsschicht 425. Hierbei weisen die Maskierungsschicht 425 und die Signalleiterschicht 120 hinsichtlich der selektiven Ablation der Maskierungsschicht 420 einen geeigneten Unterschied in Materialparametern auf.

Figuren 5A bis 5F zeigen schematische Schnittdarstellungen von mindestens Teilabschnitten einer nach einem Ausführungsbeispiel der vorliegenden Erfindung hergestellten Gassensorvorrichtung in verschiedenen Herstellungszuständen bei Ausführung einer Variante eines Herstellungsverfahrens. Bei der Gassensorvorrichtung handelt es sich beispielsweise um die Gassensorvorrichtung aus Fig. 1. Das unter Verwendung der Figuren 5A bis 5F veranschaulichte Herstellungsverfahren ist dem unter Verwendung der Figuren 2A bis 2G veranschaulichte Herstellungsverfahren ähnlich.

Fig. 5A zeigt hierbei eine schematische Schnittdarstellung eines Sensorgrundkörpers 110 der Gassensorvorrichtung. Dabei ist der Sensorgrundkörper 100 nach einem Schritt des Bereitstellens des Sensorgrundkörpers 110 des Herstellungsverfahrens gezeigt. Der Sensorgrundkörper 110 weist das Halbleitersubstrat 112 mit dem Kavitätsabschnitt 114 und die Festelektrolytschicht 116 auf. Die Festelektrolytschicht 116 ist beispielsweise als eine partiell freiliegende bzw. freistehende Yttrium-stabilisierte Zirconoxid-Elektrolytmembran bzw. YSZ-Elektrolytmembran ausgeführt.

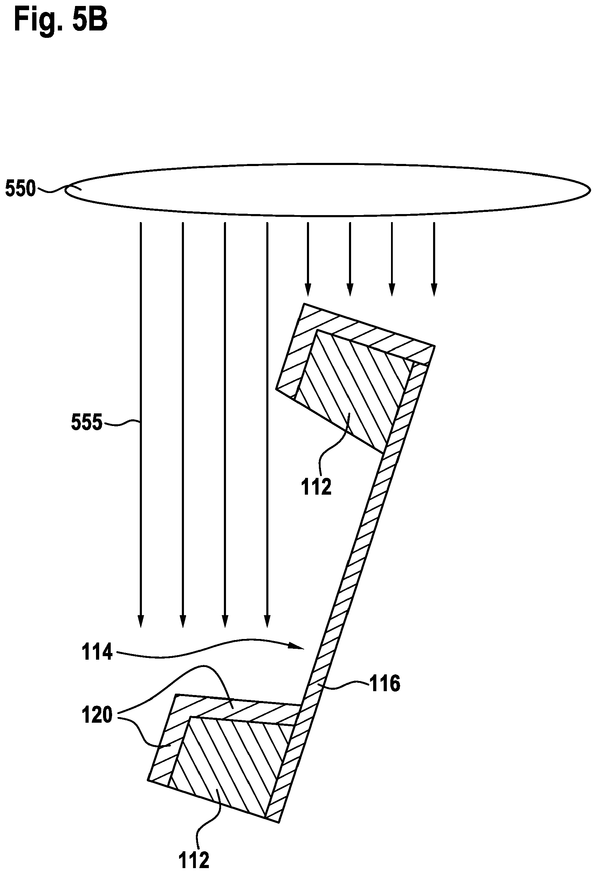

Fig. 5B zeigt eine schematische Schnittdarstellung des Sensorgrundkörpers aus Fig. 5A bei Ablauf einer Abscheidung der Signalleiterschicht 120. Hierbei ist der Sensorgrundkörper benachbart zu einer Materialquelle 550, beispielsweise einem so genannten Target, eines Materials der Signalleiterschicht 120 angeordnet. Hierbei ist eine Haupterstreckungsebene der Festelektrolytschicht 116 bzw. des Sensorgrundkörpers quer zu einer Abscheiderichtung 555 bzw. Depositionsrichtung einer Abscheidung des Materials der Signalleiterschicht 120 von der Materialquelle 550 angeordnet. Anders ausgedrückt ist in Fig. 5B genau gesagt eine gerichtete, trockenchemische Platin-Beschichtung des Sensorgrundkörpers mit der Signalleiterschicht 120 dargestellt. Dabei wird die Signalleiterschicht 120 an Teilabschnitten des Halbleitersubstrats 112 abgeschieden. Ein in dem Kavitätsabschnitt 114 freiliegender Abschnitt der Festelektrolytschicht 116 ist dabei von dem in der Abscheiderichtung 555 abgeschiedenen Material der Signalleiterschicht 120 abgeschattet bzw. geometrisch abgeschirmt. Diese geometrische Abschirmung ergibt sich aus einer Ausrichtung des Sensorgrundkörpers bezüglich der Materialquelle 550, der Abscheiderichtung 555 sowie geometrischen Eigenschaften des Kavitätsabschnitts 114.

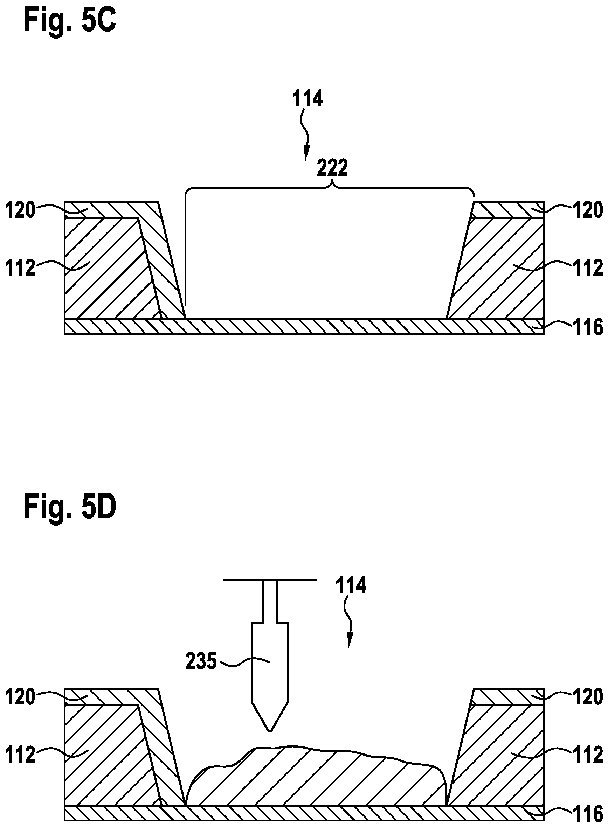

Fig. 5C zeigt eine schematische Schnittdarstellung des Sensorgrundkörpers aus Fig. 5B mit abgeschiedener Signalleiterschicht 120. Somit ist hierbei der Sensorgrundkörper in einem Herstellungszustand gezeigt, in dem die Signalleiterschicht 120 mittels trockenchemischer Abscheidung, insbesondere mittels trockenchemischer Platin-Beschichtung, in Teilabschnitten des Halbleitersubstrats 112, mehrheitlich an der Halbleitersubstratseite des Sensorgrundkörpers erzeugt ist. Hierbei ist die Signalleiterschicht 120 in Teilabschnitten des Halbleitersubstrats 112 abgeschieden, die mehrheitlich an der Halbleitersubstratseite des Sensorgrundkörpers angeordnet sind. Genau gesagt ist hierbei die Signalleiterschicht 120 so abgeschieden, dass der in dem Kavitätsabschnitt 114 freiliegende Abschnitt der Festelektrolytschicht 116 frei von der Signalleiterschicht 120 ist. Insbesondere ist hierbei in der Schnittdarstellung beispielhaft lediglich eine Flanke des Kavitätsabschnitts 114 mit der Signalleiterschicht 120 bedeckt. Somit ist in der Signalleiterschicht 120 ein Aussparungsabschnitt 222 erzeugt, in dem die Signalleiterschicht 120 unterbrochen, ausgespart bzw. unabgeschieden ist. Der Aussparungsabschnitt 222 erstreckt sich über den in dem Kavitätsabschnitt 114 freiliegenden Teil der Festelektrolytschicht 116 sowie beispielhaft über eine von dem Material der Signalleiterschicht 120 ungedeckte Flanke des Kavitätsabschnitts 114. Der Kavitätsabschnitt 114 ist somit als eine Kaverne mit Seitenwand-Platin-Film ausgeführt. Anders ausgedrückt ist die Gassensorvorrichtung in Fig. 5C in einem teilgefertigten Zustand gezeigt.

Fig. 5D zeigt eine schematische Schnittdarstellung der teilgefertigten Gassensorvorrichtung aus Fig. 5C mit einem aufgebrachten Elektrodenmaterial 230 für eine Messelektrode. Dabei ist das Elektrodenmaterial 230 in dem Aussparungsabschnitt auf die Festelektrolytschicht 116 aufgebracht. Genau gesagt ist das Elektrodenmaterial 230 in dem Aussparungsabschnitt der Signalleiterschicht 120 nasschemisch auf die Festelektrolytschicht 116 aufgebracht. Bei dem Elektrodenmaterial 230 handelt es sich beispielsweise um ein platinhaltiges Material, insbesondere eine Platin-Nanopartikellösung. Das nasschemische Aufbringen des Elektrodenmaterials 230 erfolgt hierbei zum Beispiel durch Dispensen mittels eines Aufbringwerkzeugs 235.

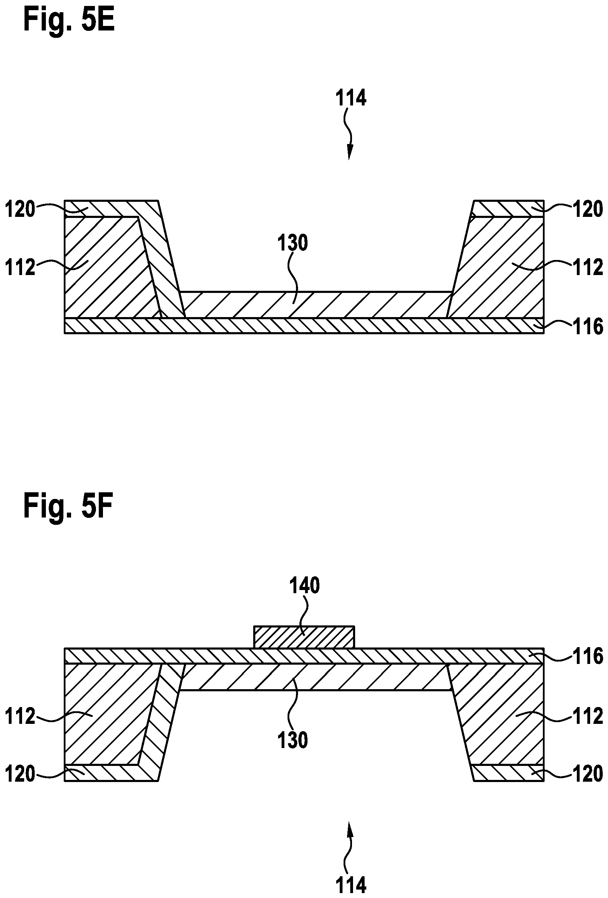

Fig. 5E zeigt eine schematische Schnittdarstellung der teilgefertigten Gassensorvorrichtung aus Fig. 5D mit gesintertem Elektrodenmaterial bzw. hergestellter erster Messelektrode 130. Somit ist in Fig. 5E die teilgefertigte Gassensorvorrichtung in einem Zustand dargestellt, in dem das Elektrodenmaterial aus Fig. 5D durch Sintern verfestigt ist und somit die erste Messelektrode 130 erzeugt ist. Die erste Messelektrode 130 ist in dem Aussparungsabschnitt der Signalleiterschicht 120 an der Festelektrolytschicht 116 angeordnet.

Fig. 5F zeigt eine schematische Schnittdarstellung der Gassensorvorrichtung 100 mit erzeugter bzw. aufgebrachter zweiter Messelektrode 140 bzw. Vorderseitenelektrode. Somit ist die Gassensorvorrichtung 100 in Fig. 5F in einem Zustand gezeigt, der dem in Fig. 1 dargestellten Zustand entspricht, wobei die Gassensorvorrichtung 100 jener aus Fig. 1 ähnlich ist. Beispielsweise handelt es sich bei dem in Fig. 5F gezeigten Zustand der Gassensorvorrichtung 100 um einen hergestellten bzw. gefertigten Zustand.

Anders ausgedrückt zeigen Figuren 5A bis 5F einen Einsatz eines gerichteten Abscheideverfahrens für einen Herstellungsverfahren der Gassensorvorrichtung 100 bzw. eines MECS-Sensorelements, um eine Verwendung funktionaler, nasschemisch aufgebrachte Elektroden für eine Rückseitenkontaktierung der Festelektrolytschicht 116 bzw. Elektrolytmembran zu ermöglichen. Somit wird ein Einsatz einer kombinierten Abscheidung bestehend aus gerichteter trockenchemischer Abscheidung und anschließender nasschemischer Abscheidung ermöglicht. Dabei erfolgt beispielsweise eine gerichtete Vakuumabscheidung eines kompaktem Metallfilms, wobei der zu beschichtende Sensorgrundkörper 110 und die Abscheidungsrichtung 555 so zueinander ausgerichtet sind, dass eine Abscheidung im wesentlichen lediglich auf den Flanken des Kavitätsabschnitts 114 und auf einer Rückseite des Halbleitersubstrats 112 erfolgt. Unter anderem für gerichtete Abscheidungen in Frage kommen bevorzugt Elektronenstrahlverdampfung, mit Einschränkungen auch Sputtern, ionenstrahlunterstütztes Sputtern oder ähnliche Verfahren. Der Winkel zwischen einer Haupterstreckungsebene des Sensorgrundkörpers 110 und der Abscheiderichtung 555, bzw. wie dieselben zueinander ausgerichtet sind, hängt dabei unter anderem von einer Tiefe und/oder einem Durchmesser des Kavitätsabschnitts 114 ab. Dabei bleibt die in dem Kavitätsabschnitt 114 freiliegende Festelektrolytschicht 116 bzw. ein Kavernenboden weitgehend unbeschichtet, um einen Zugang eines Messgases mit dem zumindest einen gasförmigen Analyten zu für einen mikroelektrochemischen Sensor wichtigen Dreiphasengrenzen, die zwischen Messgas, Messelektroden 130 und 140 sowie der Festelektrolytschicht 116 angeordnet sind, nicht beispielsweise durch die Signalleiterschicht 120 bzw. eine kompakte Platin-Schicht zu blockieren. Nach Abscheidung der Signalleiterschicht 120 auf den Teilabschnitten des Halbleitersubstrats 112 bzw. den Kavernenwänden erfolgt die nasschemische Abscheidung der ersten Messelektrode 130 am Kavernenboden, wobei eine Schichtdicke eines Elektrodenfilms so gewählt wird, dass ein Kontakt zu der Seminarleiterschicht 120 an den Kavernenwänden hergestellt ist.

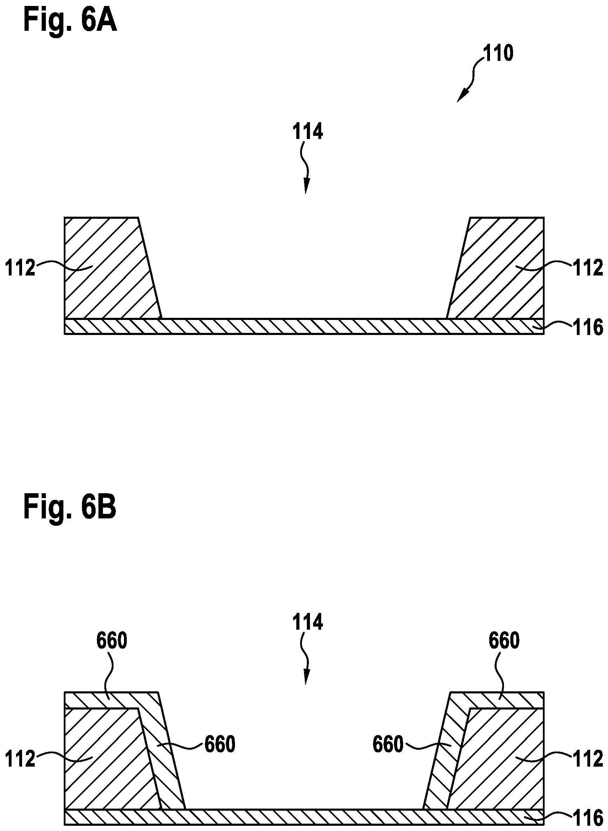

Figuren 6A und 6B zeigen schematische Schnittdarstellungen eines Sensorgrundkörpers 110 einer nach einem Ausführungsbeispiel der vorliegenden Erfindung hergestellten Gassensorvorrichtung. Dabei entspricht der in Fig. 6A gezeigte Sensorgrundkörper 110 einem der Sensorgrundkörper aus Fig. 2A und Fig. 5A. In Fig. 6B ist das Halbleitersubstrat 112 auf der Halbleitersubstratseite des Sensorgrundkörpers mit einer Passivierungsschicht 660 beschichtet. Es kann insbesondere ein beliebiger der Sensorgrundkörper 110 einer der Gassensorvorrichtungen aus Fig. 1, Figuren 2A bis 2G, Fig. 3, Figuren 4A bis 4F und Figuren 5A bis 5F mit der Passivierungsschicht 660 beschichtet sein.

Anders ausgedrückt zeigen Figuren 6A und 6B ein Bereitstellen bzw. Anordnen der Passivierungsschicht 660 auf freiliegenden Oberflächen des Halbleitersubstrats 112 zum Schutz vor Substratdegradation während eines Betriebs der Gassensorvorrichtung beispielsweise in einem Abgas. Nach dem Beschichten mit der Passivierungsschicht 660 bzw. nach einer Passivierung erfolgt beispielsweise eine Bearbeitung ähnlich oder entsprechend dem in den Figuren 5A bis 5F veranschaulichten Herstellungsverfahren, d. h. eine gerichtete Metallabscheidung zum Erzeugen einer Signalleiterschicht und ein nasschemisches Aufbringen der Messelektroden. Da ein Teil der Flanken des Kavitätsabschnitts 114 bzw. der Kavernenwände von der Signalleiterschicht unbeschichtet bleibt, kann es notwendig sein, diese unbeschichteten Bereiche mit der Passivierungsschicht 660 zu versehen, um eine Degradation der Siliziumoberfläche des Halbleitersubstrats 112 während des Sensorbetriebs, insbesondere bei Einsatz im Abgas von Verbrennungsmotoren, zu vermeiden. Für die Passivierungsschicht 660 kommen beispielsweise Oxidschichten und Nitridschichten in Frage, wie zum Beispiel Siliciumdioxid (SiO₂), Siliciumnitrid (SiN), Aluminiumoxid (AIO), Aluminiumnitrid (AIN), Titanoxid (TiO), Titannitrid (TiN) oder dergleichen. Die Passivierungsschicht 660 ist durch in der Halbleitertechnik übliche Herstellverfahren wie Sputterdeposition, Chemische Gasphasenabscheidung (CVD), Atomic Layer Deposition, Elektronenstrahlverdampfen oder Pulsed Laser Deposition (PLD) bereitstellbar. Eine einfache Methode, um die Passivierschicht 660 ausschließlich auf den ungeschützten Silizium-Flanken des Halbleitersubstrats 112 bereitzustellen, ist eine thermische Oxidation von freiliegenden Siliziumoberflächen nach einer Strukturierung bzw. nach einem Ausformen des Kavitätsabschnitts 114.

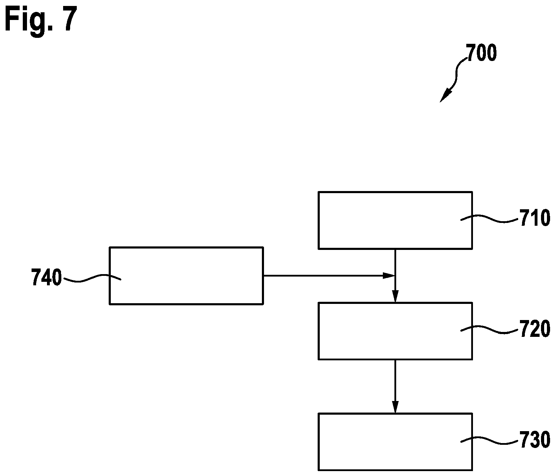

Fig. 7 zeigt ein Ablaufdiagramm eines Verfahrens 700 gemäß einem Ausführungsbeispiel der vorliegenden Erfindung. Das Verfahren 700 ist ein Verfahren zum Herstellen einer Gassensorvorrichtung. Dabei ist die Gassensorvorrichtung ausgebildet, um zumindest einen gasförmigen Analyten zu erfassen. Somit ist das Verfahren 700 ausführbar, um eine Gassensorvorrichtung wie die Gassensorvorrichtung aus einer der Figuren 1 bis 6B herzustellen. Anders ausgedrückt ist eine Gassensorvorrichtung wie die Gassensorvorrichtung aus einer der Figuren 1 bis 6B durch Ausführen des Verfahrens 700 herstellbar.

Das Verfahren 700 weist einen Schritt 710 des Bereitstellens eines Sensorgrundkörpers auf. Der Sensorgrundkörper weist dabei ein Halbleitersubstrat, in dem zumindest ein Kavitätsabschnitt ausgeformt ist, und eine an einer ersten Hauptoberfläche des Halbleitersubstrats angeordnete Festelektrolytschicht auf. Hierbei ist die Festelektrolytschicht in dem zumindest einen Kavitätsabschnitt durch das Halbleitersubstrat unbedeckt oder ausgespart.

In einem in dem Verfahren 700 nachfolgend ausführbaren Schritt 720 des Erzeugens wird eine trockenchemisch abgeschiedene Signalleiterschicht an einer Halbleitersubstratseite des Sensorgrundkörpers erzeugt, sodass im Bereich der in dem zumindest einen Kavitätsabschnitt durch das Halbleitersubstrat unbedeckten Festelektrolytschicht zumindest ein Aussparungsabschnitt in der Signalleiterschicht ausgeformt ist, in dem die Signalleiterschicht entfernt oder unabgeschieden (oder ausgespart) ist.

In einem auf den Schritt 720 des Erzeugens folgenden Schritt 730 des Aufbringens werden zumindest zwei Messelektroden auf die Festelektrolytschicht mittels eines nasschemischen Prozesses aufgebracht. Hierbei wird im Schritt 730 des Aufbringens eine erste Messelektrode in dem zumindest einen Aussparungsabschnitt der Signalleiterschicht angeordnet. Ferner wird dabei im Schritt 730 des Aufbringens eine zweite Messelektrode auf einer Festelektrolytschichtseite des Sensorgrundkörpers angeordnet.

Gemäß einem Ausführungsbeispiel wird im Schritt 720 des Erzeugens die Signalleiterschicht trockenchemisch abgeschieden und mittels selektiver Laserablation in dem zumindest einen Aussparungsabschnitt entfernt. Alternativ, aber nicht unter den Schutzumfang der Ansprüche fallend, wird im Schritt 720 des Erzeugens die Signalleiterschicht in einer definierten Abscheiderichtung trockenchemisch abgeschieden. Dabei wird in Abhängigkeit von der Abscheiderichtung und einer Geometrie des zumindest einen Kavitätsabschnitts der zumindest eine Aussparungsabschnitt vor einem Material der Signalleiterschicht abgeschattet. Optional, aber ebenfalls nicht unter den Schutzumfang der Ansprüche fallend, wird im Schritt 720 des Erzeugens die Signalleiterschicht trockenchemisch abgeschieden und mit einer Maskierungsschicht abgedeckt, wird danach die Maskierungsschicht mittels selektiver Laserablation in dem zumindest einen Aussparungsabschnitt abgetragen und wird dann die Signalleiterschicht in dem zumindest einen Aussparungsabschnitt entfernt.

Das Verfahren 700 weist gemäß einem Ausführungsbeispiel einen Schritt 740 des Beschichtens des Halbleitersubstrats mit einer Passivierungsschicht auf. Der Schritt 740 des Beschichtens wird dabei vor dem Schritt 720 des Erzeugens der Signalleiterschicht durchgeführt.

Die beschriebenen und in den Figuren gezeigten Beispiele und Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt und/oder in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (700) zum Herstellen einer Gassensorvorrichtung (100) zum Erfassen zumindest eines gasförmigen Analyten, wobei das Verfahren (700) folgende Schritte aufweist:
Bereitstellen (710) eines Sensorgrundkörpers (110), der ein Halbleitersubstrat (112), in dem zumindest ein Kavitätsabschnitt (114) ausgeformt ist, und eine an einer ersten Hauptoberfläche des Halbleitersubstrats (112) angeordnete Festelektrolytschicht (116) aufweist, wobei die Festelektrolytschicht (116) in dem zumindest einen Kavitätsabschnitt (114) durch das Halbleitersubstrat (112) ausgespart ist;
Erzeugen (720) einer trockenchemisch abgeschiedenen Signalleiterschicht (120) an einer Halbleitersubstratseite des Sensorgrundkörpers (110), sodass im Bereich der in dem zumindest einen Kavitätsabschnitt (114) durch das Halbleitersubstrat (112) unbedeckten Festelektrolytschicht (116) zumindest ein Aussparungsabschnitt (222) in der Signalleiterschicht (120) ausgeformt ist, in dem die Signalleiterschicht (120) ausgespart ist; und
Aufbringen (730) zumindest zweier Messelektroden (130, 140) auf die Festelektrolytschicht (116) mittels eines nasschemischen Prozesses, wobei eine erste Messelektrode (130) in dem zumindest einen Aussparungsabschnitt (222) der Signalleiterschicht (120) angeordnet wird und eine zweite Messelektrode (140) auf einer Festelektrolytschichtseite des Sensorgrundkörpers (110) angeordnet wird,
wobei im Schritt (720) des Erzeugens die Signalleiterschicht (120) trockenchemisch abgeschieden und mittels selektiver Laserablation in dem zumindest einen Aussparungsabschnitt (222) entfernt wird,
wobei die selektive Laserablation von der Halbleitersubstratseite des Sensorgrundkörpers (110) her ausgeführt wird und/oder von der Festelektrolytschichtseite des Sensorgrundkörpers (110) der durch die Festelektrolytschicht (116) hindurch ausgeführt wird,
wobei die Festelektrolytschicht (116) für bei der Laserablation verwendetes Laserlicht transparent ist.

2. Verfahren (700) gemäß einem der vorangegangenen Ansprüche, **dadurch gekennzeichnet, dass** im Schritt (710) des Bereitstellens ein Sensorgrundkörper (110) bereitgestellt wird, in dessen Halbleitersubstrat (112) der zumindest eine Kavitätsabschnitt (114) mit einer Tiefe von mehr als 100 Mikrometern oder mehr als 200 Mikrometern ausgeformt ist.

3. Verfahren (700) gemäß einem der vorangegangenen Ansprüche, **gekennzeichnet durch** einen Schritt (740) des Beschichtens des Halbleitersubstrats (112) mit einer Passivierungsschicht (660), wobei der Schritt (740) des Beschichtens vor dem Schritt (720) des Erzeugens der Signalleiterschicht (120) durchgeführt wird.

## Claims

1. Method (700) for producing a gas sensor device (100) for detecting at least one gaseous analyte, wherein the method (700) comprises the following steps:
providing (710) a sensor main body (110) comprising a semiconductor substrate (112), in which at least one cavity section (114) is shaped, and a solid electrolyte layer (116) arranged at a first main surface of the semiconductor substrate (112), wherein the solid electrolyte layer (116) is left free of the semiconductor substrate (112) in the at least one cavity section (114);
producing (720) a signal conductor layer (120) deposited dry-chemically at a semiconductor substrate side of the sensor main body (110), such that, in the region of the solid electrolyte layer (116) not covered by the semiconductor substrate (112) in the at least one cavity section (114), at least one cutout section (222) is shaped in the signal conductor layer (120), in which the signal conductor layer (120) is cut out; and
applying (730) at least two measuring electrodes (130, 140) to the solid electrolyte layer (116) by means of a wet-chemical process, wherein a first measuring electrode (130) is arranged in the at least one cutout section (222) of the signal conductor layer (120) and a second measuring electrode (140) is arranged on a solid electrolyte layer side of the sensor main body (110),
wherein in step (720) of producing, the signal conductor layer (120) is deposited dry-chemically and is removed by means of selective laser ablation in the at least one cutout section (222),
wherein the selective laser ablation is performed from the semiconductor substrate side of the sensor main body (110) and/or is performed from the solid electrolyte layer side of the sensor main body (110) through the solid electrolyte layer (116), wherein the solid electrolyte layer (116) is transparent to laser light used during the laser ablation.

2. Method (700) according to any of the preceding claims, **characterized in that** in step (710) of providing, a sensor main body (110) is provided, in the semiconductor substrate (112) of which the at least one cavity section (114) is shaped with a depth of more than 100 micrometers or more than 200 micrometers.

3. Method (700) according to any of the preceding claims, **characterized by** a step (740) of coating the semiconductor substrate (112) with a passivation layer (660), wherein step (740) of coating is carried out before step (720) of producing the signal conductor layer (120).

## Revendications

1. Procédé (700) de fabrication d'un dispositif de détection de gaz (100) destiné à détecter au moins un analyte gazeux, dans lequel le procédé (700) comprend les étapes consistant à :
fournir (710) un corps de base de capteur (110) qui comporte un substrat semi-conducteur (112), dans lequel est formée au moins une partie de cavité (114), et une couche d'électrolyte solide (116) disposée sur une première surface principale du substrat semi-conducteur (112), dans lequel la couche d'électrolyte solide (116) est évidée dans ladite au moins une partie de cavité (114) à travers le substrat semi-conducteur (112) ;
générer (720) une couche de conduction de signal (120) déposée par voie chimique sèche du côté substrat semi-conducteur du corps de base de capteur (110), de telle sorte que dans la zone de la couche d'électrolyte solide (116), qui n'est pas recouverte par le substrat semi-conducteur (112) dans ladite au moins une partie de cavité (114), au moins une partie évidée (222), dans laquelle la couche de conduction de signal (120) est retirée, est formée dans la couche de conduction de signal (120) ; et
appliquer (730) au moins deux électrodes de mesure (130, 140) sur la couche d'électrolyte solide (116) au moyen d'un procédé chimique par voie humide, dans lequel une première électrode de mesure (130) est disposée dans ladite au moins une partie évidée (222) de la couche de conduction de signal (120) et une seconde électrode de mesure (140) est disposée du côté couche d'électrolyte solide du corps de base de capteur (110),
dans lequel, lors de l'étape (720) de génération, la couche de conduction de signal (120) est déposée par voie chimique sèche et retirée par ablation sélective au laser dans ladite au moins une partie évidée (222),
dans lequel l'ablation sélective au laser est effectuée à partir du côté substrat semi-conducteur du corps de base de capteur (110) et/ou est effectuée à partir du côté couche d'électrolyte solide du corps de base de capteur (110) à travers la couche d'électrolyte solide (116), dans lequel la couche d'électrolyte solide (116) est transparente à la lumière laser utilisée lors de l'ablation au laser.

2. Procédé (700) selon l'une des revendications précédentes, **caractérisé en ce que** lors de l'étape (710) de fourniture, un corps de base de capteur (110) est fourni, dans le substrat semi-conducteur (112) duquel est formée ladite au moins une partie de cavité (114) avec une profondeur supérieure à 100 ou supérieure à 200 micromètres.

3. Procédé (700) selon l'une des revendications précédentes, **caractérisé par** une étape (740) de revêtement du substrat semi-conducteur (112) d'une couche de passivation (660), dans lequel l'étape (740) de revêtement est effectuée avant l'étape (720) de génération de la couche de conduction de signal (120).
